# DEMANDE DE BREVET EUROPEEN

(11) **EP 0 712 616 A1**
(43) Date de publication de la demande: **22.05.1996**
(21) Numéro de dépôt: 95402467.5
(22) Date de dépôt: 06.11.1995
(51) Int. Cl.: A61F 2/30

(54) **Fond de cotyle pour cotyles cimentés**

(30) Priorité: 17.11.1994 FR 9414063
(71) Demandeur: PROTEK SYNTHES S.A., F-25461 Etupes Cédex (FR); Vidal, Philippe, F-25300 Montbeliard (FR); Henky, Pierre, F-67115 Plobshein (FR)
(72) Inventeur: Vidal, Philippe, Cabinet Ballot-Schmit, F-25000 Besançon (FR); Henky, Pierre, Cabinet Ballot-Schmit, F-25000 Besançon (FR); Bouraly, Jean-Pierre, Cabinet Ballot-Schmit, F-25000 Besançon (FR)
(74) Mandataire: Ballot, Paul Denis Jacques

(57) **Abrégé**

Fond (10) de cotyle pour cotyles cimentés.

Selon l'invention, ledit fond de cotyle est constitué par une grille (11) métallique semi-rigide de forme sensiblement hémisphérique, destinée à être placée librement entre ledit cotyle et l'acetabulum (20) d'un patient, et portant au moins un bossage (13a,13b,13c) prévu pour coopérer avec un trou correspondant (21b) aménagé dans ledit acetabulum (20).

Application au domaine de l'arthroplastie.

## Description

La présente invention concerne un fond de cotyle pour cotyles cimentés.

L'invention trouve application dans le domaine de l'arthroplastie de la hanche mettant en oeuvre des cotyles en polyéthylène fixés dans l'acetabulum à l'aide d'un ciment acrylique.

De manière classique, l'implantation d'un cotyle dit cimenté consiste à fraiser l'acetabulum du patient pour lui donner une forme sensiblement sphérique, puis à introduire le ciment dans le fond de la cavité cotyloïdienne, et, enfin, à mettre en place le cotyle en le maintenant en position jusqu'à polymérisation complète du ciment.

Cette technique d'implantation ne permet pas toutefois d'obtenir une très bonne qualité de fixation dans le temps. En effet, le suivi clinique de ce type d'implants montre à court et moyen termes au niveau radiologique la présence de liserés osseux qui sont la preuve d'une mauvaise interface osciment. La dégradation continue du ciment conduit alors à la libération de particules qui provoquent la résorption de l'os et la désolidarisation du cotyle prothétique.

Le craquellement du ciment veilli, responsable de ce processus de descellement, a pour cause essentielle les pressions exercées et les mouvements du bassin qui induisent dans le ciment une contrainte de cisaillement résultant de la rotation de la tête de la tige fémorale à l'intérieur du cotyle de polyéthylène. Il s'ensuit une fatigue et une dégradation du ciment avec apparition de craquelures amorçant le descellement. Ce phénomène est surtout sensible dans les zones de faibles épaisseurs qui apparaissent lorsque l'implantation est effectuée sans aucun contrôle de l'homogénéité de l'épaisseur de ciment.

Pour tenter d'augmenter la longévité des cotyles cimentés, la demande de brevet européen n° 0 430 831 propose un cotyle portant sur sa surface extérieure une structure métallique ajourée fixée à des protubérances par des vis de manière à être noyée dans l'épaisseur du ciment.

La structure métallique ajourée de ce cotyle constitue une armature de renforcement pour le ciment d'ancrage dont la résistance à la rupture se trouve sensiblement améliorée. De plus, la présence de cette structure métallique évite le contact direct entre l'os et le polyéthylène.

Cependant, le cotyle connu de l'état de la technique précité présente un certain nombre d'inconvénients. En effet, il ne prévoit aucun moyen de résistance aux efforts de cisaillement dans le ciment qui reste donc très sensible aux mouvements de la tige fémorale. D'autre part, l'homogénéité d'épaisseur et de scellement obtenue n'est pas parfaite du fait que la distance grille-polyéthylène n'est pas constante et que les têtes des vis de fixation sont en contact direct avec l'os. En outre, lesdites vis de fixation étant enfoncées dans le polyéthylène le long d'un diamètre, la tête de la tige fémorale peut venir toucher l'extrémité pointue des vis à la suite de l'usure de la surface intérieure du cotyle.

Aussi, le problème technique à résoudre par l'objet de la présente invention est de réaliser un fond de cotyle pour cotyles cimentés, qui permettrait d'obtenir une réelle résistance au cisaillement dans le ciment et d'améliorer encore l'homogénéité de scellement notamment au niveau de l'épaisseur de ciment.

La solution au problème technique posé consiste, selon la présente invention, en ce que ledit fond de cotyle est constitué par une grille métallique semi-rigide de forme sensiblement hémisphérique, destinée à être placée librement entre ledit cotyle et l'acetabulum d'un patient, et portant au moins un bossage prévu pour coopérer avec un trou correspondant aménagé dans ledit acetabulum.

Avantageusement, le fond de cotyle de l'invention porte trois bossages disposés en trépied.

Lesdits bossages ont une double fonction, à savoir, d'une part, d'assurer et de maintenir le positionnement du fond de cotyle dans l'acetabulum, et, d'autre part, de s'opposer aux forces de cisaillement dont le ciment est le siège lors des mouvements du bassin.

Etant donné qu'il est complètement libre, sans aucune contrainte mécanique, le fond de cotyle conforme à l'invention se met en place de lui-même, après que le ciment et le cotyle aient été introduits, sensiblement au milieu de l'espace compris entre l'os et le cotyle, le ciment pouvant traverser le fond de cotyle grâce à la structure grillagée de ce dernier.

Le fond de cotyle, objet de l'invention, n'est donc en contact ni avec l'os ni avec le cotyle de polyéthylène, ce qui permet d'obtenir un scellement parfaitement homogène par la formation d'un véritable matériau composite très résistant présentant trois zones d'égale épaisseur : une de ciment, une de ciment et des fibres métalliques du fond de cotyle, et une de ciment.

On notera également que, du fait de son caractère semi-rigide, le fond de cotyle de l'invention peut s'adapter exactement à la forme de l'acetabulum, même lorsque celui-ci n'est pas parfaitement sphérique mais ovoïde.

Il faut de plus souligner que le fond de cotyle, objet de l'invention, n'est pas spécifique d'un cotyle donné et qu'il peut être utilisé en combinaison avec tout type de cotyles cimentés.

Enfin, un autre avantage de l'invention réside dans l'effet de diffuseur thermique produit par la grille métallique lors de la réaction de polymérisation du ciment, évitant ainsi les lésions des cellules du tissu osseux au contact avec le ciment, celui-ci pouvant atteindre une température supérieure à 60°C.

La description qui va suivre en regard des dessins annexés, donnés à titre d'exemples non limitatifs, fera bien comprendre en quoi consiste l'invention et comment elle peut être réalisée.

La figure 1 est une vue de côté en coupe d'un fond de cotyle conforme à l'invention.

La figure 2 est une vue de dessus du fond de cotyle de la figure 1.

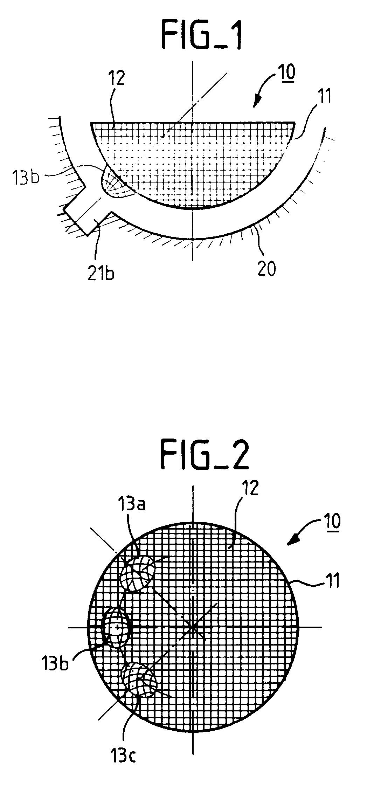

Les figures 1 et 2 montrent un fond 10 de cotyle pour cotyles cimentés, constitué par une grille 11 métallique semi-rigide de forme sensiblement hémisphérique.

Ladite grille 11 est réalisée par tissage de fils 12 d'acier inoxydable par exemple, d'un diamètre de 300µm environ et formant un maille de 450µm environ.

Le fond 10 de cotyle des figures 1 et 2 est destiné à être placé librement entre un cotyle cimenté, non représenté mais qui peut être quelconque, et l'acetabulum 20 d'un patient.

Des bossages 13a, 13b, 13c sont prévus sur la grille métallique 11 pour coopérer avec des trous correspondants, ici le trou 21b pour le bossage 13b, percés par le praticien dans l'acetabulum 20 à l'aide d'un gabarit en des endroits où l'os iliaque présente une épaisseur suffisante.

Comme on peut le voir plus particulièrement sur la figure 2, les trois bossages 13a, 13b, 13c sont disposés en trépied le long d'un même parallèle.

## Revendications

1. Fond (10) de cotyle pour cotyles cimentés, caractérisé en ce qu'il est constitué par une grille (11) métallique semi-rigide de forme sensiblement hémisphérique, destinée à être placée librement entre ledit cotyle et l'acetabulum (20) d'un patient, et portant au moins un bossage (13a,13b,13c) prévu pour coopérer avec un trou correspondant (21b) aménagé dans ledit acetabulum (20).

2. Fond de cotyle selon la revendication 1, caractérisé en ce qu'il porte trois bossages (13a,13b,13c) disposés en trépied.
